(19)

**Europäisches Patentamt**
**European Patent Office**
**Office européen des brevets**

(11) **EP 4 166 665 A1**

(12) **EUROPEAN PATENT APPLICATION**

(43) Date of publication:
**19.04.2023 Bulletin 2023/16**

(21) Application number: **21425047.4**

(22) Date of filing: **12.10.2021**

(51) International Patent Classification (IPC):
*C12N 15/10* (2006.01)   *C12P 19/30* (2006.01)

(52) Cooperative Patent Classification (CPC):
**C12N 15/1017; C12P 19/30; C12P 19/32**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Prosol S.p.A.**
**24040 Madone (BG) (IT)**

(72) Inventors:
• **BONVICINI, Daniele**
**20052 Monza (MB) (IT)**
• **LISSONI, Stefano**
**24030 Mapello (BG) (IT)**
• **TOGNI, Ermanno**
**24030 Presezzo (BG) (IT)**

(74) Representative: **Ferreccio, Rinaldo**
**Botti & Ferrari S.p.A.**
**Via Cappellini, 11**
**20124 Milano (IT)**

(54) **PROCESS FOR THE PRODUCTION OF RNA AND 5'-RIBONUCLEOTIDES WITH A HIGH
DEGREE OF PURITY**

(57)    A process for the production of **RNA** with a high purity from microbial cells, the process comprising the steps of: a) treating the cells to release the cell content thereof, including RNA; b) subjecting such cell content to an osmotic process, obtaining an RNA-enriched retentate; c) lowering the RNA-enriched retentate pH to a value comprised between 2 and 3 and recovering the precipitated RNA, obtaining a first suspension comprising precipitated RNA; d) increasing the pH of this first suspension comprising precipitated RNA to a value comprised between 4.5 and 7.5, obtaining a solution comprising solubilized RNA; e) lowering the pH of the latter solution to a value comprised between 2 and 3, and recovering the precipitated RNA, obtaining a second suspension comprising precipitated RNA; f) subjecting said second suspension comprising precipitated RNA to microfiltration, having previously increased the pH of said second suspension comprising precipitated RNA to a value comprised between 5 and 7; the present invention also relates to a process for the production of a composition comprising **5'-ribonucleotides** from microbial cells, comprising the steps of: i) treating the cells to release the cell content thereof, including RNA; ii) subjecting such cell content to an osmotic process, obtaining an RNA-enriched retentate; iii) lowering the pH of such retentate to a value comprised between 2 and 3, and recovering the precipitated RNA, obtaining a first suspension comprising precipitated RNA; iv) increasing the pH of said first suspension to a value comprised between 4.5 and 7.5, obtaining a solution comprising solubilized RNA; v) lowering the pH of such solution to a value comprised between 2 and 3, and recovering the precipitated RNA, obtaining a second suspension comprising precipitated RNA; vi) pasteurizing this second suspension at a temperature comprised between 85 °C and 95 °C, for a time period of 15-30 minutes, increasing the pH of the suspension to a value comprised between 5-5.5, obtaining a pasteurized RNA solution; vii) hydrolyzing such solution; viii) subjecting to microfiltration the hydrolyzed RNA solution obtained from step vii); ix) sterilizing the microfiltered solution of hydrolyzed RNA obtained from step viii) at a temperature comprised between 85 °C and 90 °C and performing a UV irradiation.

EP 4 166 665 A1

**Description**

<u>Application field</u>

The present invention refers to the field of food and zootechnical industry as well as the pharmaceutical industry, in particular in the areas where the consumption or the use of highly purified ribonucleic acid (RNA) is envisaged.

More particularly, the present invention relates to a process for the production of RNA and a composition comprising 5'-ribonucleotides, characterized by a high degree of purity, to be used as products of food, pharmaceutical and zootechnical interest.

<u>Background art</u>

It is known that ribonucleic acid (RNA) is formed by a sequence of nucleotides, in which the sugar is ribose, and the nitrogenous bases are adenine, guanine, cytosine, and uracil. There are numerous types of RNAs that show different levels of structural complexity, corresponding to different functions and roles within the cell; RNA can form extremely complex secondary and tertiary structures and specifically recognizes complementary sequences present both in DNA and in other RNAs.

It is also known the usefulness of ribonucleic acid in the pharmaceutical field, where it has been shown to have different potentials, including cosmetic effects (for example, reducing wrinkle thickness), energizing effects, antioxidant, and antiviral activities.

A further use of ribonucleic acid provides for its depolymerization and degradation to single ribonucleotides following enzymatic or chemical treatment. The ribonucleotides, optionally in combination with specific amino acids such as glutamate, are in fact known for their flavor enhancing properties and, for this reason, they are normally added to different types of foods, including sauces and aromatic seasonings.

Even in the zootechnical field the use of ribonucleotides is known, favoring both the correct development of younger animals and a rapid immune response against pathogens.

It is also known that primary source to obtain substantial amounts of RNA is represented by microorganisms, among which bacterial and fungal microorganisms recognized as GRAS (generally recognized as safe) by the governmental institute FDA are preferred and therefore can be used with safety for human consumption.

Previously, the Applicant proposed (WO 2017/153266) a process for the production of RNA from yeast, briefly reported herein.

After a heat treatment of yeast cells to cause the release of their cell content, including RNA, an osmotic process is performed, in particular a diafiltration, to separate the RNA from other cell soluble compounds by the use of semipermeable membranes, obtaining an RNA-enriched retentate.

Then, a salting-out step of the proteins in the RNA-enriched retentate is performed, at a pH comprised between 3.0 and 5.0.

The RNA precipitation by lowering the retentate pH to a value comprised between 2 and 3 is then determined, after that the precipitated RNA is recovered by known techniques, for example filtration or centrifugation.

Subsequently, the process further provided for an optional step of dissolving the precipitated RNA at a pH comprised between 4.5 and 7.5, removing possible impurities undissolved at such pH, and subsequently recovering RNA by repeating the aforementioned steps of precipitating RNA and recovering the precipitated RNA by.

At the end of such process, including the aforementioned optional step, the final product has the following characteristics: an UV titer of 87%, an absorbance value at 520 nm higher than 2.5, a transmittance value at 430 nm of 58.9%, and a NTU value higher than 1000.

The problem underlying the present invention was to provide a process that is particularly suitable for application on an industrial scale and that ensures an RNA and 5'-ribonucleotides production with a high degree of purity.

<u>Summary of the invention</u>

The aforesaid technical problem was solved by the present invention providing a process for the production of **RNA** from microbial cells having a high purity corresponding to a UV titer of at least 88%, the process comprising the steps of:

a) treating the cells to release the cell content thereof, including RNA;

b) subjecting the cell content comprising RNA to an osmotic process, obtaining an RNA-enriched retentate;

c) lowering the RNA-enriched retentate pH to a value comprised between 2 and 3, and recovering the precipitated RNA, obtaining a first suspension comprising precipitated RNA;

d) increasing the pH of the first suspension comprising precipitated RNA to a value comprised between 4.5 and 7.5, obtaining a solution comprising solubilized RNA;

e) lowering the pH of the solution comprising solubilized RNA to a value comprised between 2 and 3, and recovering the precipitated RNA, obtaining a second suspension comprising precipitated RNA;

f) subjecting the second suspension comprising precipitated RNA to microfiltration, having previously increased the pH of said second suspension comprising precipitated RNA to a value comprised between 5 and 7, preferably at 5.5.

Preferably, the aforesaid process comprises a step g) of subjecting to sterilization the precipitated RNA obtained from microfiltration step f).

Preferably, step a) is performed by a mechanical, enzymatic, or heat treatment, more preferably a heat treatment, wherein the temperature is comprised between 85 °C and 95 °C, even more preferably at 90 °C, for a time period comprised between 30 minutes and 120 minutes, more preferably between 40 minutes and 70 minutes.

Preferably, step b) is performed by means of membranes having a nominal cut-off comprised between 150 Da and 300 Da, said membranes being more preferably made of polysulfone.

Preferably, in step d), the increase of pH is performed by adding a basic compound to said first suspension comprising precipitated RNA, said basic compound being more preferably an alkaline or alkaline-earth hydroxide and even more preferably being selected from sodium hydroxide, calcium hydroxide, and potassium hydroxide.

Preferably, the basic compound is in the form of an aqueous solution with a concentration comprised between 20% and 40%, more preferably comprised between 25% and 35%, even more preferably with a concentration of 30%.

Preferably, step f) is performed by means of membranes having a nominal cut-off comprised between 0.2 $\mu$m and 1.2 $\mu$m, more preferably 0.45 $\mu$m, at a transmembrane pressure comprised between 0.1 bar and 6 bar, more preferably 1 bar, said membranes being even more preferably ceramic membranes.

Preferably, before step f), the second suspension comprising precipitated RNA is heated at a temperature comprised between 75 °C and 85 °C and brought to a pH comprised between 5.2 and 5.6.

Preferably, step g) is performed by heating the solution obtained from step f) at a temperature comprised between 100 °C and 120 °C, for a time period comprised between 10 and 20 minutes.

The present invention also relates to RNA from microbial cells, characterized by a UV titer of 89%, an absorbance value at 520 nm lower than 0.30, preferably comprised between 0.29 and 0.25, a transmittance value at 430 nm comprised between 85% and 90%, preferably 87%, and a nephelometric titer lower than 100 NTU, preferably comprised between 35 NTU and 40 NTU, obtained by the above-described process.

In another aspect thereof, the present invention relates to a process for the production of a composition comprising **5'-ribonucleotides** from microbial cells, comprising the steps of:

i) treating the cells to release the cell content thereof, including RNA;

ii) subjecting the RNA-comprising cell content to an osmotic process, obtaining an RNA-enriched retentate;

iii) lowering the pH of the RNA-enriched retentate to a value comprised between 2 and 3, and recovering the precipitated RNA, obtaining a first suspension comprising precipitated RNA;

iv) increasing the pH of the first suspension comprising precipitated RNA to a value comprised between 4.5 and 7.5, obtaining a solution comprising solubilized RNA;

v) lowering the pH of the solution comprising solubilized RNA to a value comprised between 2 and 3, and recovering the precipitated RNA, obtaining a second suspension comprising precipitated RNA;

vi) pasteurizing the second suspension comprising precipitated RNA at a temperature comprised between 85 °C and 95 °C, for a time period comprised between 15 minutes and 30 minutes, increasing the pH of the suspension to a value comprised between 5-5.5, obtaining a pasteurized RNA solution;

vii) hydrolyzing the pasteurized RNA solution;

viii) subjecting to microfiltration the hydrolyzed RNA solution obtained from step vii);

ix) sterilizing the microfiltered solution of hydrolyzed RNA obtained from step viii) at a temperature comprised between 85 °C and 90 °C and performing a UV irradiation.

Preferably, step i) is performed by a mechanical, enzymatic, or heat treatment, more preferably a heat treatment, wherein the temperature is comprised between 85 °C and 95 °C, even more preferably at 90 °C, for a time period comprised between 30 minutes and 120 minutes, more preferably comprised between 40 minutes and 70 minutes.

Preferably, step ii) is performed by means of membranes having a nominal cut-off between comprised 150 Da and 300 Da, said membranes being preferably made of polysulfone.

Preferably, in steps iv) and vi), the increase of pH is performed by adding a basic compound more preferably selected from an alkaline or alkaline-earth hydroxide and even more preferably being selected from sodium hydroxide, calcium hydroxide, and potassium hydroxide.

Preferably, the basic compound is in the form of an aqueous solution with a concentration comprised between 20% and 40%, more preferably comprised between 25% and 35%, even more preferably with a concentration of 30%.

Preferably, before step vii), the pasteurized RNA solution is heated at a temperature comprised between 65 °C and 75 °C and brought to a pH comprised between 5.3 and 5.5.

Preferably, the step vii) is performed enzymatically, more preferably being performed a first hydrolysis step by adding a phosphodiesterase and a second hydrolysis step by adding a deaminase, to the pasteurized RNA solution obtained from step vi).

Preferably, before step viii), the hydrolyzed RNA solution obtained from step vii) is heated at a temperature comprised between 75 °C and 85 °C.

Preferably, the microfiltration step viii) is performed by means of membranes having a nominal cut-off comprised between 0.2 $\mu$m and 1.2 $\mu$m, more preferably 0.45 $\mu$m, at a transmembrane pressure comprised between 0.1 bar and 6 bar, more preferably 1 bar, said membranes being even more preferably ceramic membranes.

Preferably, the aforementioned microbial cells are fungal, bacterial or yeast cells, more preferably a yeast belonging to a genus selected amongst *Saccharomyces* and *Kluyveromyces.*

The present invention relates also to a composition comprising 5'-ribonucleotides obtained by the above-described process, wherein the 5'-ribonucleotides are in form of heptahydrate salts, with a percentage titer higher than 60%, preferably between 65% and 70%, more preferably 68%, of the dry matter of said composition, said composition having an absorbance at 520 nm lower than 0.30, preferably comprised between 0.20 and 0.25, more preferably 0.21, and a nephelometric titer lower than 100 NTU, preferably comprised between 25 NTU and 30 NTU, more preferably 27 NTU.

The treatment according to steps a) and i), preferably a heat treatment, advantageously allows the inactivation of possible natural yeast enzymes, as well as the lysis of the cell membrane and a part of the cell wall, with the consequent leakage of the cytoplasm contained therein; thus, the RNA is extracted from the inside of the yeast cell.

Advantageously, after the aforementioned treatment, the RNA-comprising solution is separated from the exhausted yeast cells, preferably by centrifugation. The exhausted yeast cells removed from the solution result in a slurry, i.e. "exhausted yeast", which is a waste of the present process.

The thus obtained RNA-comprising solution is subjected to an osmotic process, to obtain an RNA-enriched retentate.

The steps of lowering the pH to a value comprised between 2 and 3 advantageously allow to cause the precipitation (in particular the flocculation) of the RNA molecules in the retentate or suspension in which it is contained, thus allowing the separation of these molecules from the liquid in which they are present.

Advantageously, in particular, at the end of steps c) and iii), the first suspension comprising precipitated RNA show a degree of purity defined as UV titer determined by spectrophotometer, searching the peak of maximum absorbance normally comprised between the wavelengths of 257 and 259 nm. Such UV titer of the first suspension comprising precipitated RNA is about 60-65%.

The increase of pH of steps d) and iv) allows advantageously to re-solubilize the RNA in order to release any impurities that may have remained incorporated in the RNA flocs formed during the preceding precipitation steps c) and iii).

At the end of steps e) and v), advantageously, the second suspension comprising precipitated RNA shows a degree of purity defined as UV titer determined by spectrophotometer, searching the peak of maximum absorbance normally comprised between the wavelengths of 257 and 259 nm. Such UV titer of the second suspension comprising precipitated RNA is 70-75%.

The microfiltration step f) allows advantageously to further increase the degree of purity, and therefore the quality of the final product.

Furthermore, such step has the purpose of making the solution clear; in fact, the turbidity resulting from the presence of particles with dimensions higher than those of the RNA molecules, is removed by means of membranes having a specific cut-off range.

In particular, such membrane cut-off range to carry out the microfiltration, according to the present invention, is advantageously comprised between 0.2 μm and 1.2 μm, preferably 0.45 μm.

In particular, the aforementioned membranes are ceramic membranes, more specifically of pure alumina.

Moreover, such membranes to perform the microfiltration withstand advantageously a transmembrane working pressure comprised between 0.5 bar and 6 bar; according to the present invention, the more advantageous transmembrane working pressure is 1 bar.

With reference specifically to the process to obtain a composition comprising 5'-ribonucleotides according to the present invention, the hydrolysis step vii) involves the enzymatic digestion of the solution comprising RNA obtained from step vi), by the action of two enzymes: a phosphodiesterase and a deaminase.

In particular, the phosphodiesterase enzyme allows the cleavage of the phosphodiester bonds between each single nucleotide that makes up the RNA molecules, thus obtaining a solution comprising the 5'-ribonucleotides 5'-CMP, 5'-AMP, 5'-UMP, and 5'-GMP.

The deaminase enzyme, instead, advantageously converts the 5'-AMP ribonucleotide in 5'-IMP (inosine monophosphate). The presence of 5'-IMP is advantageous as it contributes, together with 5'-GMP and glutamate (derived from glutamic acid) to the perception of umami taste. This feature of the product is appreciated by the flavoring industry, for which the product is intended.

At the end of the hydrolysis step, the pH of the solution containing hydrolyzed RNA can be advantageously brought to a value comprised between 5.9 and 6.5, conveniently 6.2, to standardize the pH of the finished product.

The subsequent microfiltration step viii) of the hydrolyzed RNA solution, comprising the aforesaid nucleotides, removes advantageously a substantial amount of the substances in solution which contribute to the opaque appearance of the same, as well as to an increase in the percentage title of 5'-ribonucleotides compared to dry matter (grams of nucleotides/ 100 g of d.m. *100).

In particular, such microfiltration step viii) takes place under the same conditions as the aforementioned microfiltration step f), *i.e.,* by means of membranes, preferably ceramic membranes, conveniently membranes of pure alumina, having a cut-off comprised between 0.2 μm and 1.2 μm, preferably 0.45 μm.

During the microfiltration step, a transmembrane working pressure comprised between 0.5 bar and 6 bar, conveniently 1 bar, is applied.

At the end of the microfiltration step, the solution comprising the RNA hydrolysate can be advantageously concentrated to remove water by the combined action of temperature and vacuum application: in these conditions, the evaporation of the water constituting the solution occurs at temperatures below 100 °C, preserving the integrity of the 5'-nucleotides.

The sterilization step ix) of the microfiltered solution of hydrolyzed RNA occurs advantageously at a temperature of 90 °C, thus preserving the structure, and therefore the functionality, of 5'-nucleotides (extremely thermolabile beyond 100 °C), and at the same time reducing and restraining a possible bacterial load present.

Finally, UV irradiation, occurring preferably in a wavelength range comprised between 200 nm and 280 nm (UV-C), conveniently at 254 nm, advantageously guarantees a further reduction of a possible bacterial load present in the product.

The RNA obtained as described in the present invention has a high degree of purity, determined by UV titer, absorbance at 520 nm, nephelometric titer, transmittance at 430 nm.

In particular, the UV titer provides an indication of the purity of the sample, and must be higher than 88%; the absorbance at 520 nm provides a colorimetric indication of the product and must be preferably lower than 0.50; the transmittance at 430 nm provides an indication of the transparency of the product and must be preferably of 87% (the pure water reading returns a value of 100%), and the nephelometric titer providing an indication of the amount of light refracted by the particles present in the solution, must be preferably lower than 100 NTU.

Advantageously, in fact, the RNA obtained according to the present invention shows a UV titer of 89%, an absorbance at 520 nm of 0.27, a nephelometric titer of 36 NTU and a transmittance at 430 nm of 87%.

The composition comprising 5'-ribonucleotides obtained according to the present invention has a high degree of purity and shows, in fact, a heptahydrate 5'-ribonucleotides titer (determined as a percentage by weight of dry matter, and preferably higher than 60%) of 68%, an absorbance at 520 nm of 0.21, and a nephelometric titer of 27 NTU.

Detailed description of a preferred embodiment

Further features and advantages of the present invention will become evident from the following examples, given herein for illustrative and nonlimiting purposes.

**EXAMPLES**

*Materials and methods*

- *Determination of purity and identification of RNA by UV method.*

Legislative references: ISO 78-2: 1999
The method is based on the maximum optical absorbance in the UV range spectrum from 250 to 290 nm of a sample solution of RNA, referred to the theoretical value A = 25000 u.u./g, with an optical path of 1 cm.

*Reagents and materials:*

- NaOH (30-33%)

- 0.1 M Phosphate buffer (pH 7)

- Water (analytic grade)

*Equipment:*

- Precision analytical balance

- Usual laboratory equipment

- UV-visible spectrophotometer

*Methods:*

2 grams of powder sample to be tested are weighed and transferred in a 1000 ml volumetric flask using water. 2 ml of NaOH (30-33%) are added, and then water up to a volume of 1000 ml is added and stirring is kept for 40 minutes (solution A). 2 ml of solution A are then transferred to a 100 ml volumetric flask containing 10 ml of 0.1 M phosphate buffer and diluted to volume with water.
The solution is analyzed by spectrophotometer using water as blank: absorbance at 250 nm, 260 nm, 280 nm, 290 nm, and maximum absorbance are determined.
Also, the transmittance value at a 430 nm on "solution A" is recorded.

*Calculations:*

$$\text{UV titer (\%RNA)} = [(A_{max} / 25000 \text{ x } 100 / 2 \text{ x } 1000) / \text{weight}] \text{ x } 100$$

- *Color intensity and turbidity of RNA in aqueous solution.*

*Legislation*: ISO 78-2: 1999
This method allows to determine the color and the turbidity of a sample solution.
A 2% RNA or hydrolyzed RNA solution (pH 5.6 - 6.0) is considered acceptable in turbidity and color if its absorbance value at 520 nm is lower than 0.9 and the turbidity value with nephelometric system is lower than 100 NTU.
In particular, optimal values are lower than 0.3 for absorbance and lower than 50 NTU for turbidity.
Values comprised between 0.3 and 0.9 for color absorbance and between 50 and 100 for NTU show a beginning of a "cloudy" aspect.

*Reagents and materials:*

- Water (analytic grade)

- 1 N Sodium hydroxide

*Equipment:*

- Precision analytical balance

- Usual laboratory equipment

- UV-visible spectrophotometer

*Methods:*

About $2 \pm 0.05$ g of the powder sample to be tested are weighed and transferred in a 250 ml beaker.
100 ml of analytic grade water are added.
The sample is left under stirring until complete dissolution.
Some drops of sodium hydroxide are added if the pH of the solution is lower than 5.6.
The solution is analyzed by spectrophotometer using water as blank.

*Calculations:*

Recording of the absorbance values obtained at 520 nm, recorded with UV spectrophotometer equipment; recording of the NTU values provided by "Merck TURBIQUANT 3000 IR" equipment.

- *Quantification of 5-ribonucleotides by HPLC.*

*Legislation*: ISO 78-2: 1999
This method allows to determine the 5'-ribonucleotides amount in a sample, using an external reference standard.

*Reagents and materials:*

During the analysis, unless otherwise stated, only reagents of recognized analytical grade, and water for HPLC were used.

Mobile phase:

A: $NaH_2PO_4$ buffer

B: Acetonitrile

Equipment:

- analytical precision balance;

- HPLC system with quaternary pump, VWD detector, auto-sampler, column thermostat and degasser;

- Column C18, 250 x 4.6 mm-5 microns.

*Procedure:*

The HPLC column was previously conditioned with the mobile phases A for at least 60 minutes at a 1 ml/min flow rate.

The gradient for the GRAD-RIG method was the following:

| Time (minutes) | %A | % B |
|---|---|---|
| 0 | 100 | 0 |
| 15 | 100 | 0 |
| 30 | 70 | 30 |
| 35 | 70 | 30 |
| 40 | 20 | 80 |
| 45 | 20 | 80 |
| 46 | 100 | 0 |
| 55 | 100 | 0 |

End time: 57 minutes

Flow rate: 1.0 ml/min

Column temperature: 25 °C

Wavelenght: 260 nm

Injection volume: 10 $\mu$L

In these conditions, the retention time of 5'-ribonucleotides was the following:

CMP: 5 minutes

UMP: 6 minutes

AMP: 9 minutes

IMP: 11 minutes

GMP: 13 minutes

Standard solution: an amount of 25 mg of each standard was weighted and diluted in 100 ml of HPLC grade water. After the solubilization, 10 ml of each solution is diluted in 100 ml of HPLC grade water.

Sample solution: an amount of about 30 mg of sample was weighted and diluted in 100 ml of HPLC grade water.

*Calculations:*

The final report was printed at the end of the analyses, wherein the peaks and the relative areas were reported. The retention time of the standard and the sample was then compared; the difference in retention time must not be higher than 5% absolute.

For the calculation of the content of each ribonucleotide, the following formula was used:

$$\text{Area}_{standard} : \text{concentration}_{standard} = \text{area}_{sample} : \text{concentration}_{sample}.$$

The result was expressed as the percentage ratio between the nucleotides concentration in mg/L obtained from the aforementioned formula and the sample solution concentration, also expressed in mg/L.

**Example 1** - **Method for production of RNA with high degree of purity**

*- Extraction step*

In a production reactor with a total volume of 10000 kg, 5000 kg of osmotic water were loaded, to which were added 1600 kg of powdered inactivated yeast of the genus *Kluyveromyces.* Alternatively, it is also possible to use 1600 kg of powdered inactivated yeast of the genus *Saccharomyces.*

The reactor was externally insulated and equipped with a flowing steam heating system.

The temperature of the water and inactivated yeast suspension was brought to 90 °C and kept for 1 hour.

The thus obtained solution was cooled at 75-80 °C by the addition in the reactor of 1000-1200 Kg cold osmotized water.

By passing in series on two separating centrifuges Westfalia, HFB and HFC respectively equipped with nozzles of 2 mm (HFB) and 2.5 mm (HFC), the exhausted yeast cells were removed from the solution resulting in a slurry in an amount of about 6000-6500 kg, having a dry matter normally comprised between 18 and 22%.

Separately, to the solution containing the yeast extract and RNA an amount of water was then added up to the total volume of 13000 L, thus obtaining a solution having a dry matter value of 3.5% and a pH comprised between 5 and 5.5.

*- Osmosis step*

The solution obtained from the extraction step was then sent to the supply tanks of the osmosis system.

Such plant is equipped with 27 polysulfone membranes having a nominal cut-off of 150-300 Da, mod. DK8038C, and it is programmed to perform a concentration treatment in a 1:2 ratio on the incoming liquid. The waste permeate contained an amount of chloride ions, phosphate ions, potassium ions, sodium, and sulphates.

At the end of the osmosis step, it was obtained an about 6000-6500 L volume of retentate comprising RNA and having a dry matter content comprised between 5 and 5.5%; further, a volume of about 6000-6500 L of waste permeate was obtained.

*- Purification steps*

The RNA comprising retentate, obtained from the osmosis step, was then sent back to the production department, at a temperature of about 45-50 °C and a pH around 4.3-4.5.

It was then subjected to a cooling process, by passing over four exchangers with decreasing temperatures, and then stored in a designated tank at a temperature of 4 °C - 6.5 °C.

The first RNA purification step was performed as follows.

By the addition of 92 L of a 30% HCl aqueous solution, the retentate pH was lowered to a value of about 2, thus causing the precipitation, by flocculation, of the RNA contained in the retentate.

Using clarifiers Reda Mod. RD80 and Reda Mod. RD130, the flocculated RNA was separated, thus obtaining 1800 L of first suspension comprising precipitated RNA, at 6% of dry matter, and 5600 L of waste liquid.

After this first purification step, the RNA quality is measurable as about 60-65% by means of a spectrophotometer, indicating the UV titer determined searching for the peak of maximum absorbance normally comprised between the wavelengths of 257 and 259 nm.

The first suspension comprising precipitated RNA, obtained from the first purification step, was subjected to a second purification step, according to the following steps.

In particular, the first suspension comprising precipitated RNA was diluted in a 1:1 ratio with osmotized water and the pH of the thus obtained solution was adjusted to 6 by adding 23 L of a 30% NaOH aqueous solution.

The thus obtained RNA-comprising solution was kept under stirring and at a controlled temperature comprised between 3 °C and 7 °C for at least 30 minutes.

The solution was then subjected to a precipitation step, lowering the pH to 2 by adding 28 L of a 30% HCl aqueous solution.

The thus acidified solution was subjected to a further RNA flocculation using the aforementioned clarifiers, thus obtaining about 950 kg of RNA-containing solution, having a dry matter on average of 9-10%, and 3000-3500 L of waste liquid having a dry matter of 1.6%, of which the 5% consisting of proteins.

At the end of the second purification step, a second suspension comprising precipitated RNA was then obtained.

The quality of the RNA obtained from the second purification step, expressed as UV titer, has reached an average UV titer of 70-75%.

*- Microfiltration, osmosis and sterilization steps*

The second suspension comprising precipitated RNA, obtained from the aforementioned second purification step, was subjected to a microfiltration step.

The plant, made by Reda SPA, contained a filtering apparatus consisting of 99 tubular ceramic membranes of 0.45 $\mu$m, (in particular, of pure alumina material), having a length of 1000 mm, a diameter of 35 mm, and produced by GENERAL ELECTRIC.

In particular, the microfiltration step provided for heating the RNA-comprising suspension at a temperature of about 80 °C, and increasing the solution pH to values comprised between 5.2-5.6, by adding 30% NaOH aqueous solution.

Subsequently, the RNA-comprising solution was pumped to the microfiltration membranes; against a given volume of liquid permeated by the membranes (liquid containing purified RNA), the supply tank with the liquid still to be treated (added with the liquid retentate back from the membranes) was replenished of equal volume with osmotized water. The transmembrane pressure set during the microfiltration step was 1 bar.

A diafiltration process was then carried out, performed, and continued until the reintegration of 3 times the starting volume (starting volume in the supply tank), thus resulting in a diafiltration ratio 3.

At the end of the diafiltration process, the microfiltration step was performed until the supply liquid runs out, *i.e.,* the suspension comprising precipitated RNA obtained from the second purification step.

At the end of the microfiltration step, the RNA-containing solution was subjected to an osmosis step on 8 membranes model RO8038/30. The working temperature in this step is 45 °C.

By means of this osmosis step, the water supplied with the diafiltration was removed, with a concentration factor of 10, until having a dry matter in solution of 18%. The concentration treatment with osmosis membranes does not affect the performance of the process.

At the end of the osmosis step, 435 L of purified RNA in solution with 18% of dry matter, a mass yield of the microfiltration of 85%, and a dry matter loss of 5% were obtained.

The RNA-containing solution was then collected in a production reactor, capable of being heated up to 120 °C, for the sterilization step at 120 °C for 15 minutes.

After this heat treatment, the RNA-containing solution was cooled to 90 °C and subjected to drying (spray dryer).

At the end of the process according to the present invention, 78.3 kg of powdered RNA were obtained.

The qualitative evaluation of such an RNA was performed based on four analytical parameters and the reference limit values thereof:

1. UV titer >88%

2. Absorbance at 520 nm <0.30

3. Transmittance at 430 nm: 87%

4. Nephelometric titer <100 NTU

The process was validated by four industrial productions. The average of the results obtained is shown in Table 1:

Table 1: Average of values of UV titer, absorbance at 520 nm, nephelometric titer, and transmittance at 430 nm of the RNA obtained according to the process of Example 1 of the present invention.

| UV titer | 92% |
|---|---|
| Absorbance at 520 nm | 0.27 |
| Nephelometric titer | 36 NTU |
| Transmittance at 430 nm | 87% |

The yield of the present process was calculated as the percentage ratio between the amount (in kg) of powdered RNA at the end of the process and the amount (in kg) of used yeast:

$$\frac{78.3 \text{ kg (amount of powdered RNA)}}{1600 \text{ kg (amount of yeast)}} * 100 = 4.9\%$$

**Example 2** - **Method for production of a composition comprising 5'-ribonucleotides with high degree of purity**

*- Hydrolisis step*

To obtain the composition comprising 5'-ribonucleotides with high degree of purity, according to the present invention, 8700 kg of powdered inactivated yeast of the genus *Kluyveromyces* were subjected to the same extraction, osmosis, and purification steps as above described in Example 1.

500 kg of RNA-comprising suspension obtained from the second purification step, as described in Example 1, were in aqueous solution with a concentration comprised between 8% and 19% of dry matter (corresponding to a volume of 5000 - 6250 L), and were transferred to a hydrolysis reactor, equipped with heating by passage of steam in the jacket.

The pH of the solution was adjusted to a value of 5-5.5 by adding 30 L of a 30% NaOH aqueous solution.

Subsequently, the solution was subjected to a pasteurization step at 90 °C for 20 minutes.

The solution was then cooled by adding cold water, up to an overall volume comprised between 12000 and 13000 L.

A second heating step at 70 °C was performed, followed by a subsequent correction of the pH to values comprised between 5.3 and 5.5, by adding 35 L of a 30% NaOH aqueous solution, thus obtaining the optimal experimental conditions for performing the subsequent hydrolysis step by enzymes.

The hydrolysis step consisted of a first step of enzymatic digestion by a phosphodiesterase (Enzyme Flavorpro™ 848MDP, supplied by Byocatalysts Limited) at a dosage of 0.33% on d.m. in solution, for a time period comprised between 7 hours and 10 hours.

The second step of the hydrolysis is an enzymatic digestion by a deaminase (Enzyme Flavorpro™ 954MDP, supplied by Byocatalysts Limited), at a dosage of 0.44% on d.m. in solution, for a time period comprised between 1 hour and 3 hours.

At the end of the hydrolysis step, the pH of the solution was brought to 6.0-6.5 by adding a 30% NaOH aqueous solution.

*- Microfiltration and concentration step*

Subsequently, the hydrolyzed RNA solution was subjected to a microfiltration step.

In particular, after heating the solution at a temperature of 75 °C, the liquid solution was pumped to the microfiltration membranes having a cut-off of 0.45 μm; against a given volume of liquid permeated by the membranes (purified liquid containing nucleotides), the supply tank with the liquid still to be treated (added with the liquid retentate back from the membranes) was replenished of equal volume with osmotized water.

A diafiltration process was then performed, in automatic mode until the reintegration of 0.8 times the starting volume (starting volume in the supply tank); therefore, the diafiltration ratio is 0.8.

The volume of water for the diafiltration is therefore of 13000L*0.8 = 10400 L.

The step of passage on ceramic membranes was followed by an osmosis treatment on No. 8 membranes model RO8038/30, removing the water supplied with the diafiltration, with a concentration factor 3, until having a dry matter in solution of 5.5%. The working temperature in this step was 45 °C. The concentration treatment with osmosis membranes did not affect the performance of the process.

The solution obtained from osmosis was treated on a HTE tube bundle concentrator, which allows to remove water due to the combined action of heating and vacuum application (-830 millibar). Such concentrator consists of three stages of progressive concentration working respectively at 75 °C, 70 °C and 56 °C.

The machine therefore allows the evaporation of water at temperatures lower than the traditional 100 °C, preserving the integrity of the 5'-ribonucleotides.

From the concentration step, 1500 L of solution containing RNA hydrolysate having a content of dry matter of 28%-29% were obtained.

The yield of the microfiltration step was 85% on a dry basis.

*- Concentration, pasteurization, UV treatment and drying steps.*

The solution from the microfiltration step was treated on a tube bundle concentrator, obtaining a solution having a dry matter comprised between 26% and 30%, and consequently with a final volume of about 1600 L.

The thus obtained concentrated solution was stored in a reactor, which served as a supply tank for the spray system. The temperature of the reactor, and therefore of the concentrated solution, was kept constant a 90 °C.

On the power line that connected the aforementioned reactor to the spray drying system, a UV irradiation chamber was activated (as the liquid passed through).

The technical characteristics of the chamber were the following: manufacturer Montagna s.r.l., model M8S WCS MAN, power 0.41 kW. The irradiating system consists of 8 UV lamps (254 nm), contained in relative tubes of ultra-pure quartz.

A powdered RNA hydrolysate of a weight of 427 kg was obtained.

The qualitative evaluation of the product obtained at the end of the process was performed based on three analytical parameters and the reference values thereof:

| 1. Titer of heptahydrate nucleotides, % on d.m. | >60% |
| 2. Absorbance at 520 nm | <0.30 |
| 3. Nephelometric titer | <100 NTU |

The process was validated by three industrial productions. The average of the results obtained is shown in Table 2:

Table 2: Average of the values of the titer of heptahydrate nucleotides, absorbance and nephelometric titer of the RNA hydrolysate obtained by the Example 2 of the present invention.

| Titer of heptahydrate nucleotides, % on d.m. | 68% |
| --- | --- |
| Absorbance at 520 nm | 0.21 |
| Nephelometric titer | 27 NTU |

The yield of the present process was calculated as the percentage ratio between the amount (in kg) of powdered RNA hydrolysate at the end of the process and the used amount (in kg) of yeast:

$$\frac{427 \text{ kg (amount of RNA hydrolized)}}{8700 \text{ kg (amount of yeast)}} * 100 = 4.9\%$$

## EXAMPLE 3 - Comparative test

The Applicant performed a comparative test between a composition of 5'-ribonucleotides obtained according to Example 2 of the present invention (Sample A), and a composition of 5'-ribonucleotides obtained according to the same Example 2 wherein, however, the microfiltration step was not carried out (Sample B).

In particular, regarding the preparation of Sample B, following the hydrolysis step of the pasteurized RNA solution, the hydrolyzed RNA solution is immediately sterilized and subjected to UV treatment according to the operating conditions of Example 2.

The comparative test compared the quality of Sample A and Sample B, determining in particular the titer of heptahydrate 5'-ribonucleotides (in percentage on dry matter), the absorbance values at 520 nm, and the nephelometric titer.

The results are reported in the following Table 3.

Table 3. Quality comparison of Sample A and Sample B.

| | Sample A (according to Example 2 of the present invention) | Sample B |
| --- | --- | --- |
| Titer of heptahydrate nucleotides, % on d.m. | 68% | 47% |
| Absorbance at 520 nm | 0.21 | > 1.0 |
| Nephelometric titer | 27 NTU | > 700 NTU |

The results clearly show that Sample A, obtained according to the present invention, has a higher quality than Sample B.

In fact, not only Sample A shows a nucleotide titer higher than Sample B, but is shows a degree of purity higher than Sample B (as determined by the absorbance and nephelometric titer values).

**Claims**

1. A process for the production of **RNA** from microbial cells having a high purity corresponding to a UV titer of at least 88%, the process comprising the steps of:

   a) treating said cells to release the cell content thereof, including RNA;
   b) subjecting said cell content comprising RNA to an osmotic process, obtaining an RNA-enriched retentate;
   c) lowering the said RNA-enriched retentate pH to a value comprised between 2 and 3, and recovering the precipitated RNA, obtaining a first suspension comprising precipitated RNA;
   d) increasing the pH of said first suspension comprising precipitated RNA to a value comprised between 4.5 and 7.5, obtaining a solution comprising solubilized RNA;
   e) lowering the pH of said solution comprising solubilized RNA to a value comprised between 2 and 3, and recovering the precipitated RNA, obtaining a second suspension comprising precipitated RNA;
   f) subjecting said second suspension comprising precipitated RNA to microfiltration, having previously increased the pH of said second suspension comprising precipitated RNA to a value comprised between 5 and 7, preferably at 5.5.

2. The process according to claim 1, comprising a step g) of subjecting to sterilization the precipitated RNA obtained from said microfiltration step f).

3. The process according to claim 1 or 2, wherein said step a) is performed by a mechanical, enzymatic, or heat treatment, preferably a heat treatment, wherein the temperature is comprised between 85 °C and 95 °C, preferably at 90 °C, for a time period comprised between 30 minutes and 120 minutes, preferably between 40 minutes and 70 minutes.

4. The process according to any one of claims 1-3, wherein said step b) is performed by means of membranes having a nominal cut-off comprised between 150 Da and 300 Da, said membranes being preferably made of polysulfone.

5. The process according to any one of claims 1-4, wherein in said step d), the increase of pH is performed by adding a basic compound to said first suspension comprising precipitated RNA, said basic compound being preferably an alkaline or alkaline-earth hydroxide and more preferably being selected from sodium hydroxide, calcium hydroxide, and potassium hydroxide.

6. The process according to claim 5, wherein said basic compound is in form of an aqueous solution with a concentration comprised between 20% and 40%, preferably comprised between 25% and 35%, more preferably with a concentration of 30%.

7. The process according to any one of claims 1-6, wherein said step f) is performed by means of membranes having a nominal cut-off comprised between 0.2 $\mu$m and 1.2 $\mu$m, preferably 0.45 $\mu$m, at a transmembrane pressure comprised between 0.1 bar and 6 bar, preferably 1 bar, said membranes being preferably ceramic membranes.

8. The process according to any one of claims 1-7, wherein before said step f), said second suspension comprising precipitated RNA is heated at a temperature comprised between 75 °C and 85 °C, and brought to a pH comprised between 5.2 and 5.6.

9. The process according to any one of claims 1-8, wherein said step g) is performed by heating the solution obtained from said step f) at a temperature comprised between 100 °C and 120 °C, for a time period comprised between 10 and 20 minutes.

10. RNA from microbial cells, **characterized by** an UV titer of 89%, an absorbance value at 520 nm lower than 0.30, preferably comprised between 0.29 and 0.25, a transmittance value at 430 nm comprised between 85% and 90%, preferably 87%, and a nephelometric titer lower than 100 NTU, preferably comprised between 35 NTU and 40 NTU, obtained by the process according to any one of claims 1-9.

11. A process for the production of a composition comprising **5'-ribonucleotides** from microbial cells, comprising the steps of:

    i) treating said cells to release the cell content thereof, including RNA;

ii) subjecting said RNA-comprising cell content to an osmotic process, obtaining an RNA-enriched retentate;

iii) lowering the pH of said RNA-enriched retentate to a value comprised between 2 and 3, and recovering the precipitated RNA, obtaining a first suspension comprising precipitated RNA;

iv) increasing the pH of said first suspension comprising precipitated RNA to a value comprised between 4.5 and 7.5, obtaining a solution comprising solubilized RNA;

v) lowering the pH of said solution comprising solubilized RNA to a value comprised between 2 and 3, and recovering the precipitated RNA, obtaining a second suspension comprising precipitated RNA;

vi) pasteurizing said second suspension comprising precipitated RNA at a temperature comprised between 85 °C and 95 °C, for a time period comprised between 15 minutes and 30 minutes, increasing the pH of the suspension to a value comprised between 5-5.5, obtaining a pasteurized RNA solution;

vii) hydrolyzing said pasteurized RNA solution;

viii) subjecting to microfiltration said hydrolyzed RNA solution obtained from said step vii);

ix) sterilizing the microfiltered solution of hydrolyzed RNA obtained from step viii) at a temperature comprised between 85 °C and 90 °C, and performing a UV irradiation.

12. The process according to claim 11, wherein said step i) is performed by a mechanical, enzymatic, or heat treatment, preferably a heat treatment, wherein the temperature is comprised between 85 °C and 95 °C, preferably at 90 °C, for a time period comprised between 30 minutes and 120 minutes, preferably comprised between 40 minutes and 70 minutes.

13. The process according to claim 11 or 12, wherein said step ii) is performed by means of membranes having a nominal cut-off comprised between 150 Da and 300 Da, said membranes being preferably made of polysulfone.

14. The process according to any one of claims 11-13, wherein in said steps iv) and vi), the increase of pH is performed by adding a basic compound preferably selected from an alkaline or alkaline-earth hydroxide and more preferably being selected from sodium hydroxide, calcium hydroxide, and potassium hydroxide.

15. The process according to claim 14, wherein said basic compound is in form of an aqueous solution with a concentration comprised between 20% and 40%, preferably comprised between 25% and 35%, more preferably with a concentration of 30%.

16. The process according to any one of claims 11-15, wherein before said step vii), the pasteurized RNA solution is heated at a temperature comprised between 65 °C and 75 °C and brought to a pH comprised between 5.3 and 5.5.

17. The process according to any one of claims 11-16, wherein said step vii) is performed enzymatically, preferably being performed a first hydrolysis step by adding a phosphodiesterase and a second hydrolysis step by adding a deaminase, to said pasteurized RNA solution obtained from step vi).

18. The process according to any one of claims 11-17, wherein before said step viii), the hydrolyzed RNA solution obtained from step vii) is heated at a temperature comprised between 75 °C and 85 °C.

19. The process according to any one of claims 11-18, wherein said microfiltration step viii) is performed by means of membranes having a nominal cut-off comprised between 0.2 $\mu$m and 1.2 $\mu$m, preferably 0.45 $\mu$m, at a transmembrane pressure comprised between 0.1 bar and 6 bar, preferably 1 bar, said membranes being preferably ceramic membranes.

20. The process according to any one of claims 1-9 and 11-19, wherein said microbial cells are fungal, bacterial or yeast cells, preferably a yeast belonging to a genus selected from *Saccharomyces* and *Kluyveromyces*.

21. A composition comprising 5'-ribonucleotides obtained by the process according to any one of claims 11-20, wherein said 5'-ribonucleotides are in form of heptahydrate salts, with a percentage titer higher than 60%, preferably between 65% and 70%, more preferably 68%, of the dry matter of said composition, said composition having an absorbance at 520 nm lower than 0.30, preferably comprised between 0.20 and 0.25, more preferably 0.21, and a nephelometric titer lower than 100 NTU, preferably comprised between 25 NTU and 30 NTU, more preferably 27 NTU.

Europäisches
Patentamt

European
Patent Office

Office européen
des brevets

## EUROPEAN SEARCH REPORT

**Application Number**

EP 21 42 5047

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP S51 106791 A (SANYO KOKUSAKU PULP CO) 21 September 1976 (1976-09-21) <br><br> * the whole document * <br> ----- | 1-3,5,6, 8-12, 14-18, 20,21 | INV. <br> C12N15/10 <br> C12P19/30 |
| X | EP 3 426 792 A1 (PROSOL S P A [IT]) 16 January 2019 (2019-01-16) <br><br> * the whole document * <br> ----- | 1-6, 8-18,20, 21 | |
| A | US 4 623 723 A (KELLER REINHOLD [DE] ET AL) 18 November 1986 (1986-11-18) <br> ----- | 1-21 | |
| A | WO 2004/067758 A2 (DSM IP ASSETS BV [NL]; NOORDAM BERTUS [NL]; KORTES JAN GERRIT [NL]) 12 August 2004 (2004-08-12) <br> ----- | 1-21 | |
| A | US 4 649 111 A (KELLER REINHOLD [DE] ET AL) 10 March 1987 (1987-03-10) <br> ----- | 1-21 | |

**TECHNICAL FIELDS SEARCHED (IPC)**

C12N
C12P

The present search report has been drawn up for all claims

| Place of search | Date of completion of the search | Examiner |
|---|---|---|
| Munich | 24 March 2022 | Bassias, Ioannis |

EPO FORM 1503 03.82 (P04C01)

**EP 4 166 665 A1**

## ANNEX TO THE EUROPEAN SEARCH REPORT
## ON EUROPEAN PATENT APPLICATION NO.

EP 21 42 5047

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2022

| Patent document cited in search report | | Publication date | Patent family member(s) | | Publication date |
|---|---|---|---|---|---|
| JP S51106791 | A | 21-09-1976 | JP | S5724117 B2 | 22-05-1982 |
| | | | JP | S51106791 A | 21-09-1976 |
| EP 3426792 | A1 | 16-01-2019 | EP | 3426792 A1 | 16-01-2019 |
| | | | WO | 2017153266 A1 | 14-09-2017 |
| US 4623723 | A | 18-11-1986 | AT | 28751 T | 15-08-1987 |
| | | | DE | 3308932 A1 | 13-09-1984 |
| | | | DK | 94084 A | 13-09-1984 |
| | | | EP | 0121778 A1 | 17-10-1984 |
| | | | JP | S59173094 A | 29-09-1984 |
| | | | US | 4623723 A | 18-11-1986 |
| WO 2004067758 | A2 | 12-08-2004 | AT | 455859 T | 15-02-2010 |
| | | | AU | 2004207932 A1 | 12-08-2004 |
| | | | BR | PI0406903 A | 03-01-2006 |
| | | | CA | 2513590 A1 | 12-08-2004 |
| | | | CN | 101155929 A | 02-04-2008 |
| | | | CN | 102524737 A | 04-07-2012 |
| | | | DK | 1587947 T3 | 10-05-2010 |
| | | | EP | 1587947 A2 | 26-10-2005 |
| | | | JP | 4786525 B2 | 05-10-2011 |
| | | | JP | 2006525794 A | 16-11-2006 |
| | | | KR | 20050100378 A | 18-10-2005 |
| | | | PT | 1587947 E | 29-03-2010 |
| | | | US | 2006078972 A1 | 13-04-2006 |
| | | | WO | 2004067758 A2 | 12-08-2004 |
| | | | ZA | 200505433 B | 31-05-2006 |
| US 4649111 | A | 10-03-1987 | AT | 24019 T | 15-12-1986 |
| | | | AU | 551838 B2 | 15-05-1986 |
| | | | CA | 1191102 A | 30-07-1985 |
| | | | DE | 3136940 A1 | 31-03-1983 |
| | | | EP | 0075262 A2 | 30-03-1983 |
| | | | ES | 8306163 A1 | 01-05-1983 |
| | | | JP | S5867192 A | 21-04-1983 |
| | | | KR | 840001593 A | 07-05-1984 |
| | | | RO | 84708 B | 30-09-1984 |
| | | | SU | 1435158 A3 | 30-10-1988 |
| | | | US | 4649111 A | 10-03-1987 |
| | | | ZA | 826796 B | 27-07-1983 |

EPO FORM P0459

For more details about this annex : see Official Journal of the European Patent Office, No. 12/82

**EP 4 166 665 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017153266 A